# EUROPEAN PATENT SPECIFICATION

(11) **EP 1 040 797 B1**
(45) Date of publication and mention of the grant of the patent: **03.01.2007**
(21) Application number: 00302527.7
(22) Date of filing: 28.03.2000
(51) Int. Cl.: A61F 9/01

(54) **Corneal surgery apparatus**
Einrichtung zur Chirurgie der Hornhaut
Dispositif pour la chirurgie cornéenne

(30) Priority: 31.03.1999 JP 9380999
(43) Date of publication of application: 04.10.2000
(73) Proprietor: Nidek Co., Ltd., Gamagori-shi, Aichi 443-0035 (JP)
(72) Inventor: Nakamura, Takua, Hoi-gun, Aichi 441-0321 (JP)
(74) Representative: Jones, David Colin

(56) References cited:
- DE-C- 19 702 335
- US-A- 5 735 276
- US-A- 5 800 424

## Description

### BACKGROUND OF THE INVENTION

### 1.Field of the Invention

The present invention relates to a corneal surgery apparatus, which irradiates a cornea of a patient's eye with a laser beam and ablates it to correct a refractive error or to remove a lesion.

### 2.Description of Related Art

Conventionally known technique is a corneal surgery apparatus that ablates a corneal epithelium or a corneal stroma of a patient's eye by using the laser beam such as a rare gas halide excimer laser (especially, 193nm wavelength of ArF laser beam) to change a refractive power of the cornea thereby correcting refractive errors including myopia, hypermetropia and astigmatism, or to remove a corneal lesion such as superficial corneal opacity or granular corneal dystrophy.

In laser beam irradiation methods of this kind of apparatus, a slit scan method has been suggested: it irradiates a cornea with a laser beam whose beam vertical cross section is rectangular when irradiating and shows generally uniform energy intensity distribution in a longitudinal direction of the rectangular cross section (a horizontal direction) and shows gaussian distribution in an orthogonal direction relative to the longitudinal direction (a vertical direction) in a manner that the laser beam is being overlapped in the gaussian distribution direction (as disclosed in US 5,507,799 corresponding to JP. HEI 4-242644A). Further, as disclosed in US 5,637,109 (corresponding to JP. HEI 6-114083A) a method of rotating a scanning direction of a rectangular laser beam at predetermined angle intervals such as 120° has been suggested for ablating cross section uniformly.

As other irradiation methods, a one-shot irradiation method has been suggested, which irradiates an ablation range (area) of a cornea with one large laser beam, and a flying spot (spot scan) method, which converges a laser beam into a small spot by a focusing lens and scans the laser beam at a high speed as swinging the laser beam with a galvano-mirror along X and Y axes.

However, energy intensity distribution of the laser beam in a direction of beam vertical cross section can be different from the expected results due to diffusion characteristics (divergence) of the laser beam and characteristics of optical members disposed in the laser irradiation system. For example, the energy intensity distribution is generally uniform when emitting, but it is non-uniform on a cornea. The energy intensity distribution decreases in a peripheral portion relative to a center portion. Especially, in the slit scan method mentioned above, the energy intensity distribution in the longitudinal direction can be generally uniform when emitting and non-uniform on a cornea. Accordingly, when this kind of laser beam is irradiated as scanning, ablation depth of a peripheral portion is insufficient compared to that of a center of an ablation range. In the method of rotating a scanning direction of the laser beam, ablation depth of whole peripheral portion is also insufficient compared to ablation depth of the center portion. The center of laser beam whose energy intensity distribution is generally uniform can only be used. However, efficiency falls off because of small laser diameter.

In the flying spot method, the laser beam irradiates the cornea at a certain angle relative to a principal optical axis (a standard optical axis) of the irradiation optical system and the angle of irradiation becomes greater as the laser beam passes farther away from the principal optical axis. Because of this, compared to the peripheral portion of the ablation range, ablation depth of the peripheral portion is also insufficient. Further, the ablation depth may be insufficient by a curved shape of cornea and the insufficient ablation depth also happen in all methods such as the scan method, the one-shot irradiation method and the flying spot method.

The insufficient ablation depth of the peripheral portion in the ablation range widens corneal curvature. A hyperopic shift of the refractive power may occure after surgery compared to the refractive power may occur before surgery. The refractive power after surgery may be more hyperopic compared to the expected refractive power after surgery. Especially, it has a big effect on phototherapeutic keratectomy (PTK) that ablates the cornea uniformly to remove a lesion and myopic astigmatism correction.

Additionally, the laser beam of the energy intensity distribution which is different from the expected results scans in a manner to being overlapped in the fixed direction. The ablated cross section is not uniform than expected by overlapping the laser irradiation position in the same position.

US-A-5 800 424 discloses an apparatus for use in operating upon a cornea of an eye comprising a light delivery optical system for delivering an ultraviolet laser beam emitted from a laser source onto the cornea, a diaphragm with an aperture, disposed in the light delivery optical system, for restricting an irradiation area of the laser beam, a device for shifting the laser beam with respect to an optical axis of the light delivery optical system, a device for rotating the laser beam about the optical axis of the light delivery optical system at each shifting position to ablate the cornea circularly, a device for inputting information necessary for determining the shape of the post operation cornea, a device for determining ablation amount at each shifting position of the laser beam by the beam shifting device, based on the information input through the input device, and a device for controlling the laser source and action of the beam rotating device based on the ablation amount determined by the ablation amount determining device at each shifting position, wherein the laser beam ablates the cornea of the eye to correct ametropia including hypermetropia

### SUMMARY OF THE INVENTION

The present invention has been made in view of the above circumstances and has an object to overcome the above problem and to provide a corneal surgery apparatus which complements the insufficient ablation depth of a peripheral portion in the ablation range caused by a laser characteristic, a laser irradiation method and a shape of the cornea, and prevents occurrences of hyperopic shift of a cornea after ablation.

A further object of the invention is to provide a corneal surgery apparatus which can ablate the ablated cross section uniformly when the energy intensity distribution of the laser beam is different from the expected results.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

To achieve the objects and in accordance with the purpose of the invention, as embodied and broadly described herein, a corneal surgery apparatus for ablating a cornea of a patient's eye with a laser beam comprises laser irradiation means including a laser light source and an irradiating optical system for guiding the laser beam emitted from the laser light source and irradiating the cornea with the laser beam, input means for inputting required surgery conditions which determines a required shape of a cornea after ablation, and a control unit which is connected to the laser irradiation unit and the input means for controlling the laser irradiation unit in such a way to irradiate the laser beam in non-uniform irradiation frequency within an ablation range based on the inputted surgery conditions.

### BRIEF DESCRIPTION OF THE DRAWINGS

The accompanying drawings, which are incorporated in and constitute a part of this specification illustrated embodiments of the invention and together with the description, serve to explain the objects, advantages and principles of the invention. In the drawings:
Fig.1 is a schematic view showing an optical system and a control system of an apparatus of the present invention;
Fig.2 is a view illustrating energy intensity distribution of a laser beam at the time of emission;
Fig.3 is a view illustrating energy intensity distribution of the laser beam on a cornea;
Figs.4A-4F are views illustrating conventional scanning and rotating methods;
Figs.5A and 5B are views illustrating occurrences of a hyperopic shift which can be caused by the conventional scanning and rotating methods;
Figs.6A and 6B are views illustrating scanning and rotating way in accordance with the invention;
Figs.7A-7E are views illustrating scanning and rotating way in accordance with the invention;
Fig.8 is a view illustrating rotation of a scanning direction of the laser beam;
Fig.9 is a view illustrating shifts of an irradiation position of a laser beam;
Figs. 10A and 10B are views illustrating a method of shifting an irradiation position of a laser beam; and
Figs. 11A and 11B are views illustrating an example of modification of scanning and rotating ways in accordance with the invention.

### DETAILED DESCRIPTION OF THE PREFEERRED EMBODIMENTS

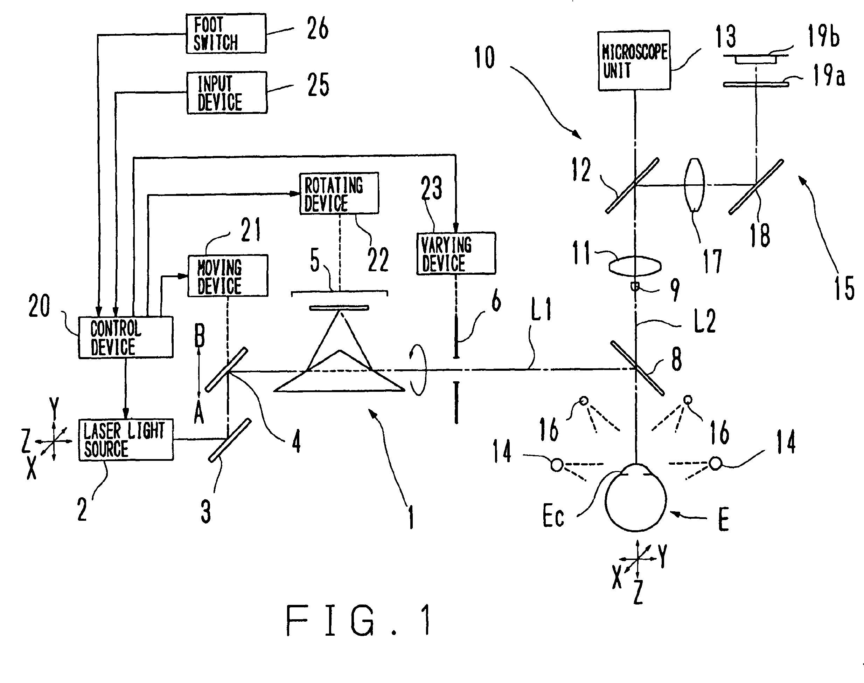

A detailed description of one preferred embodiment of a corneal surgery apparatus embodying the present invention will now be given referring to the accompanying drawings. Fig.1 is a schematic view showing an optical system and a control system of the corneal surgery apparatus of the present invention.

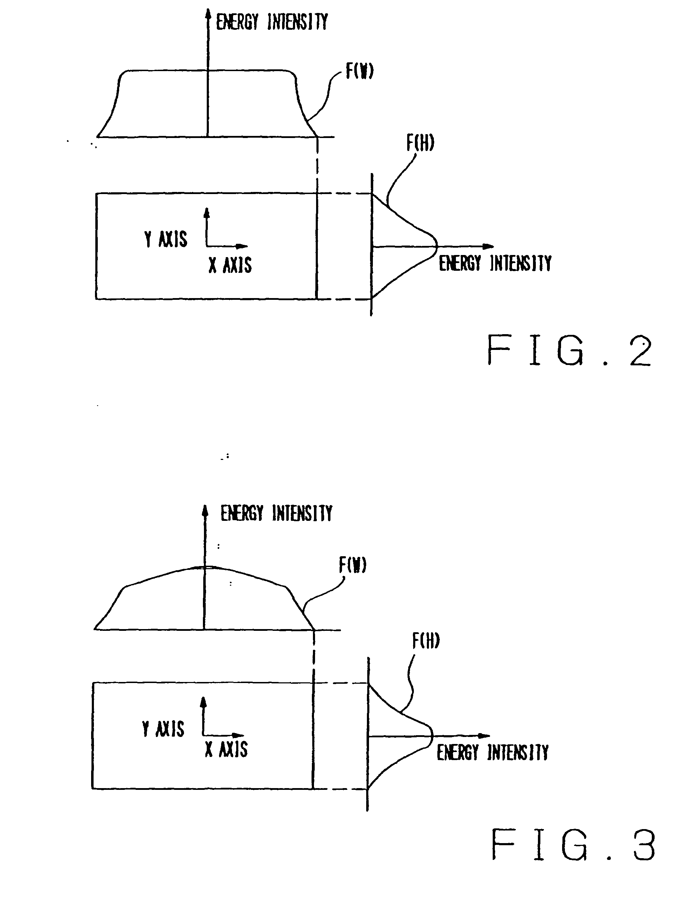

Reference numeral 1 is an irradiating optical system which guides the laser beam to a cornea Ec of a patient's eye E and irradiates it with the laser beam. The laser source 2 emits an excimer laser (ArF laser) beam whose wavelength is 193nm with pulsed oscillation. The beam vertical cross section of the laser beam emitted from the laser source 2 is rectangular. The energy intensity distribution shows generally uniform distribution F(W) in a longitudinal direction of the rectangular cross section (X axis direction) and shows the gaussian distribution F(H) in an orthogonal direction (Y axis direction)relative to the X axis direction (See Fig. 2).

Reference numerals 3 and 4 are plane mirrors. The laser beam emitted horizontally from the laser source 2 is deflected upward with the mirror 3 and again deflected horizontally at 90 ° with the mirror 4. The mirror 4 is moved parallel in an arrow (A-B) direction of Fig. 1 with a moving device 21 and scans the laser beam in a direction of the gaussian distribution (describe in detail hereinafter).

Reference numeral 5 is an image rotator which rotates a scanning direction of the laser beam with the mirror 4 about the principal axis L1 of the irradiating optical system 1. The image rotator 5 is rotated at the fixed speed and in the fixed direction with a rotating device 22.

As for the moving device 21 or the rotating device 22, these devices can use well- known mechanism such as a motor, a driver controlled by the control device 20 described hereinafter which drives the motor, a cam, a guide and a holder for moving the mirror 4 and rotating the image rotator 5 with the motor.

6 is an aperture for restricting the ablation range (area) with the laser beam. The diameter of the aperture is varied by a varying device 23 (The aperture 6 may be disposed just in front of a cornea Ec).

A dichroic mirror 8 makes the principal axis L1 of the irradiating optical system 1 and the optical axis L2 of an observation optical system 10 and a eye ball position detecting optical system 15 coaxially. The dichroic mirror 8 reflects the excimer laser beam and transmits the visible light and the near-infrared light. The observation optical system 10 comprises an objective lens 11, a dichroic mirror 12, a binocular microscope unit 13 and a visible light source 14. This allows an examinee to observe the eye E. The detecting optical system 15 comprises a near-infrared light source 16, a photographing lens 17, a mirror 18, a near-infrared light transmission filter 19a and a CCD camera 19b for the near-infrared. This detecting optical system 15 is provided to facilitate alignment of the eye E and the principal axis L1 (the axis L2) in directions of up and down or right and left (direction of X and Y axes). This allows an examinee to perform auto alignment and auto tracking. The explanation of the auto alignment and auto tracking is omitted since they have less to do with the present invention. For the detailed descriptions of auto alignment and auto tracking, see EP 0765648 corresponding to JP. HEI 9-149914 and HEI 10-192333 by the present applicant. An index projecting optical system projects two slit indexes for alignment in directions of back and forth onto the cornea Ec. It is disposed in the observation optical system 10 (unillustrated). The explanation thereof is omitted since this also has less to do with the present invention. For the detailed description of this, see US 5,562,656 corresponding to JP. HEI 6-47001. A fixation light 9 is disposed on the optical axis L2.

The control device 20 such as CPU controls the light source 2, the moving device 21, the rotating device 22, the varying device 23 and the like. Reference numeral 25 is an input device for inputting surgery conditions such as the ablation diameter (ablation range) or the ablation depth (amount of ablation). A foot switch 26 transmits a trigger signal for laser irradiation.

Hereinafter, operation of the apparatus having configurations as mentioned above will be described. In the present embodiment, phototherapeutic keratectomy (PTK) is described as an example.

First, the surgery conditions are inputted by the input device 25. In the case of PTK in the present embodiment, the ablation diameter (the ablation range) and the ablation depth (the amount of ablation) relative to the cornea Ec and the like is inputted by the input device 25. The control device 20 controls the varying device 23 based on the inputted ablation diameter and varies opening diameter of the aperture 6 and further, determines the number of scans of the laser beam based on the inputted ablation depth. In the present embodiment, the repetition frequency of the laser pulses emitted from the light source 2 can be determined between 5-50 Hz.

Next, the examiner first makes the patient lie down on an unillustrated bed. The examiner makes the patient fix the fixation light 9 to fix the eye E. The examiner observes the eye E with the microscope unit 13 and aligns it in directions of up and down and right and left, or in directions of back and forth using the index projecting optical system. At alignment, using the auto alignment and the auto tracking with the detecting optical system 15 mentioned before, the eye E and the apparatus are positioned easily. The examiner can choose whether he use the auto alignment and the auto tracking by an illustrated switch.

After completion of positioning of the eye E and the apparatus, the trigger signal is transmitted by the foot switch 26. The control device 20 controls the light source 2 based on the inputted trigger signal and oscillates laser pulses at the determined repetition frequency. The control device 20 controls the moving device 21 and moves the mirror 4 parallel, and scans the laser beam at a speed taking account of the determined repetition frequency in a direction of gaussian distribution. The control device 20 also controls the rotating device 22. The rotating device 20 synchronizes the image rotator 5 with laser pulses at the fixed speed (180° per pulse). As the image rotator 5 rotates at 180°, the scanning direction of the laser beam rotates at 360°.

After movement of the mirror 4 completes one scan of the laser beam, the control device 20 controls the light source 2 to momentarily delay the laser emission. The device rotates a scanning direction of the laser beam in the next scan at the amount of predetermined angle relative to a direction of the laser beam in the former scan. It moves the mirror 4 through the moving device 21 and scans the laser beam. A scan of the laser beam and the rotation of a scanning direction is carried out repeatedly to ablate the cornea Ec at the ablate inputted depth.

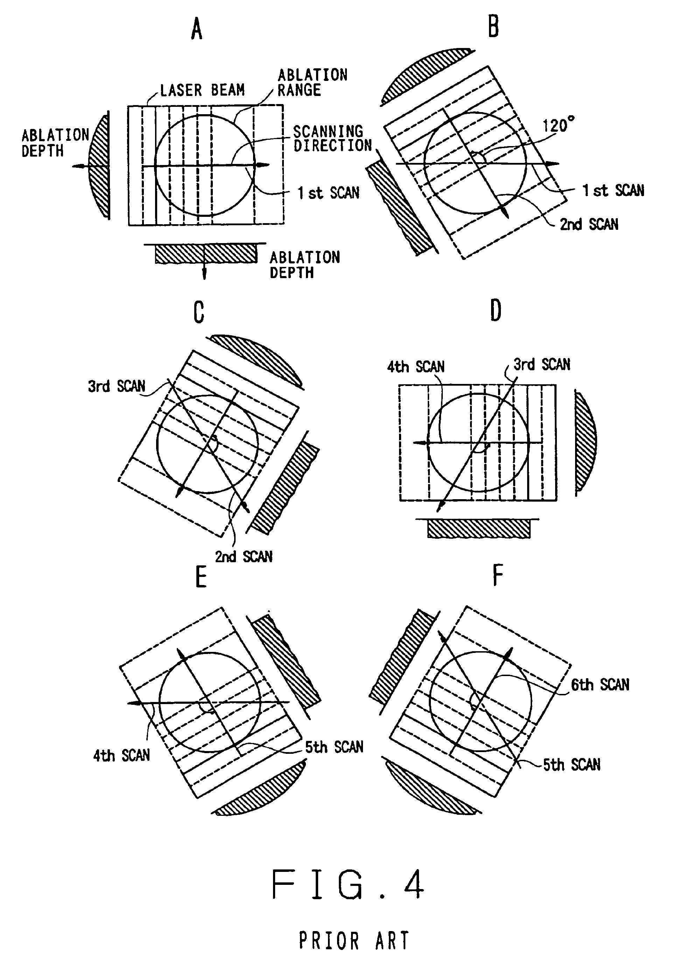

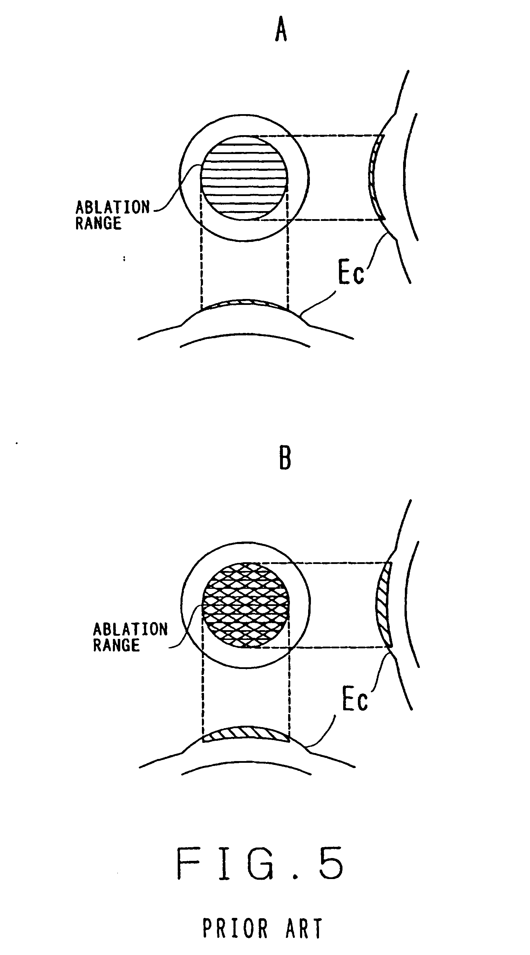

Hereinafter, a scan of the laser beam and rotation of a scanning direction will be described in detail. The energy intensity distribution of the laser beam in the longitudinal direction of the rectangular cross section shows generally uniform energy intensity distribution when emitted from the light source 2 (see Fig. 2) and shows non-uniform energy intensity distribution on a cornea Ec (see Fig.3) because of divergence of the laser beam and the like. Accordingly, as indicated in Fig.4A, after completion of the first scan, the ablation depth of a peripheral portion is shallow compared to the center portion. A hyperopic shift of the cornea Ec occurs in one direction (an orthogonal direction relative to a scanning direction) (see Fig.5A). As indicated in Figs.4 B-F, in case that the scanning direction of the laser beam is rotated at every predetermined angle to be overlapped with the second, third, fourth, fifth and sixth scan, the whole peripheral portion becomes shallow compared to the center portion. A hyperopic shift occurs in the cornea Ec in all directions (see Fig.5B). This kind of hyperopic shift can be caused by a method of the laser irradiation or a shape of a cornea.

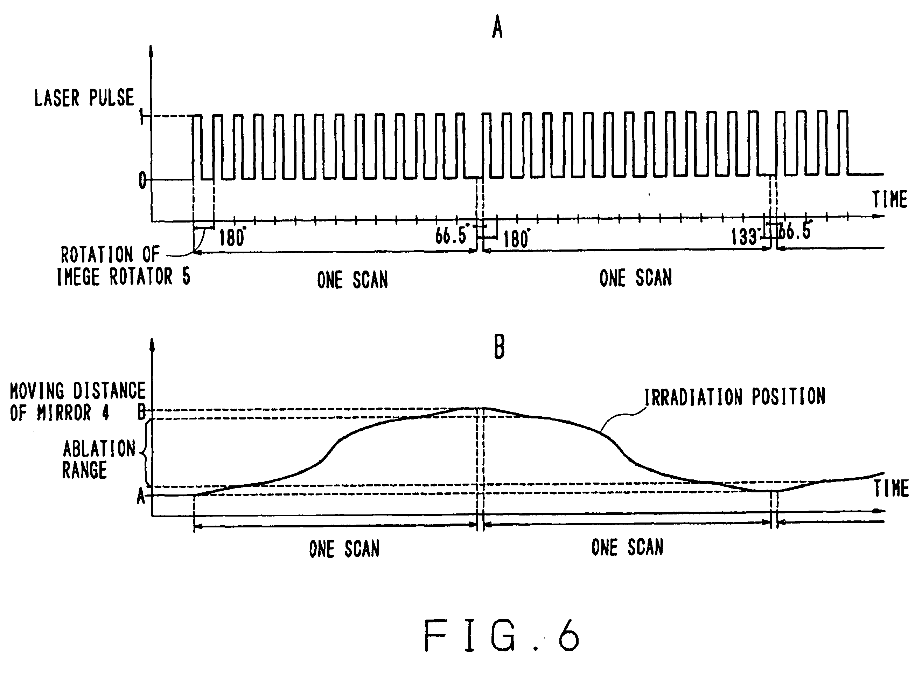

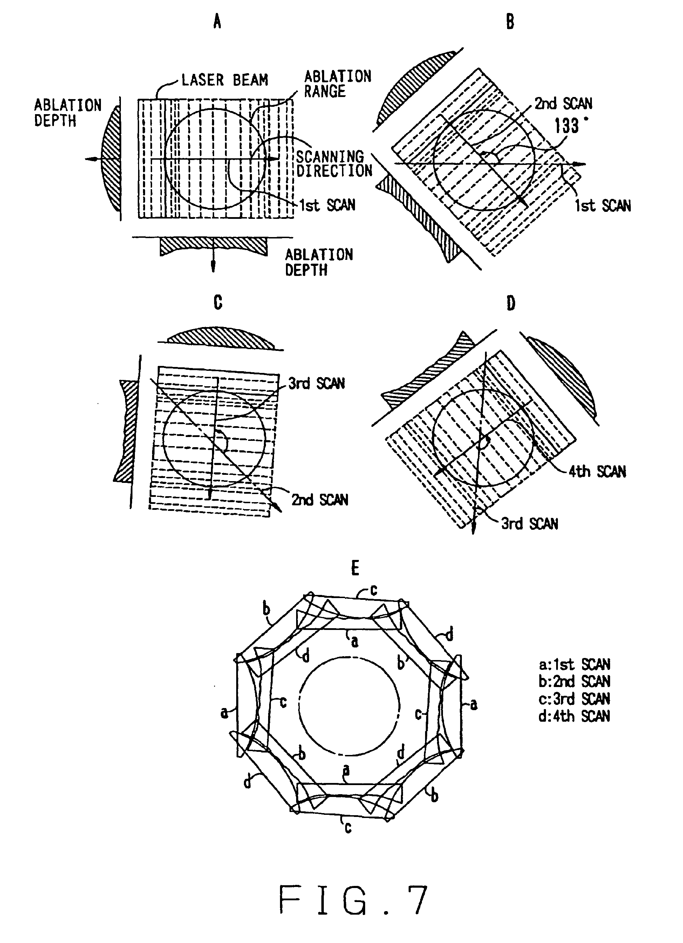

In the apparatus of the present invention, variation of moving speed of the mirror 4 in one scan and rotation of a scanning direction at every predetermined angle prevents shallow ablation in the peripheral portion. That is, the moving speed of the mirror 4 after a start and before completion of scanning in one scan is delayed (see Fig. 6B) relative to the laser pulse emission at the same interval based on the determined repetition frequency (see Fig. 6A), the number of irradiation of the laser beam is increased (the irradiation interval becomes frequent) in the peripheral portion. When the first scan is completed, the ablation depth is not uniform in the center and peripheral portion (see Fig.7A). However, the center and peripheral portion can be generally uniform by rotating a scanning direction at predetermined angle (133° in Fig.7) to be overlapped with the second, third, and forth scan (see Figs.7B-E). In the case of successive oscillation of the laser beam, not pulsed oscillation, the moving speed of the mirror 4 in one scan is varied to extend the irradiation time of the laser beam after a start and before completion of scanning in one scan. In this case, as rotating the image rotator 5 at every scan, during rotation, the laser beam is blocked by a shutter and the like (or the laser source itself may be controlled).

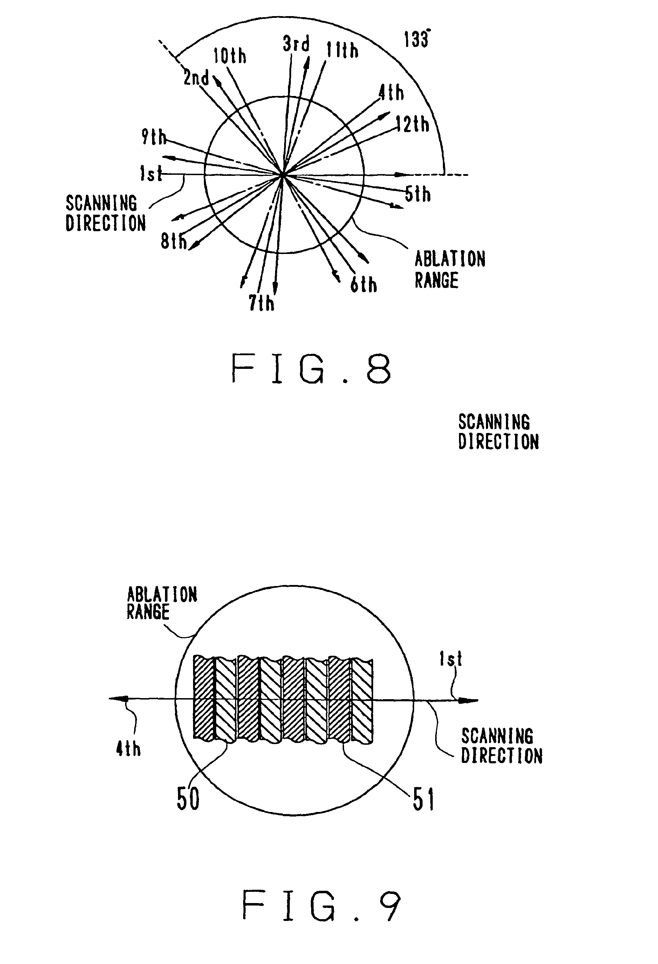

Additionally, in the present embodiment, the scanning direction of the laser beam is rotated at 133° per scan (rotation of the image rotator 5 is of 66.5° per scan). This provides more uniform ablation since the scanning direction is not the same direction (a scan path) even if the number of scans is increased (see Fig.8). Rotation angle of the scanning direction is not necessary at 133°, the unillustrated switch in the inputted device 25 may change determination. As for the rotation angle of the scanning direction, it is preferable that the determined angle is the different scanning direction (scan path) even if the number of scans is increased, and also increases dispersion of the angle with less number of variations of the scanning direction such as 133° or 227°. It is also preferable that the angles are overlapped less when performing scans in many times. For rotation of the scanning direction at a predetermined angle, in addition to the way to shift the timing of the laser irradiation as mentioned before, the rotation speed of the image rotator 5 may be varied.

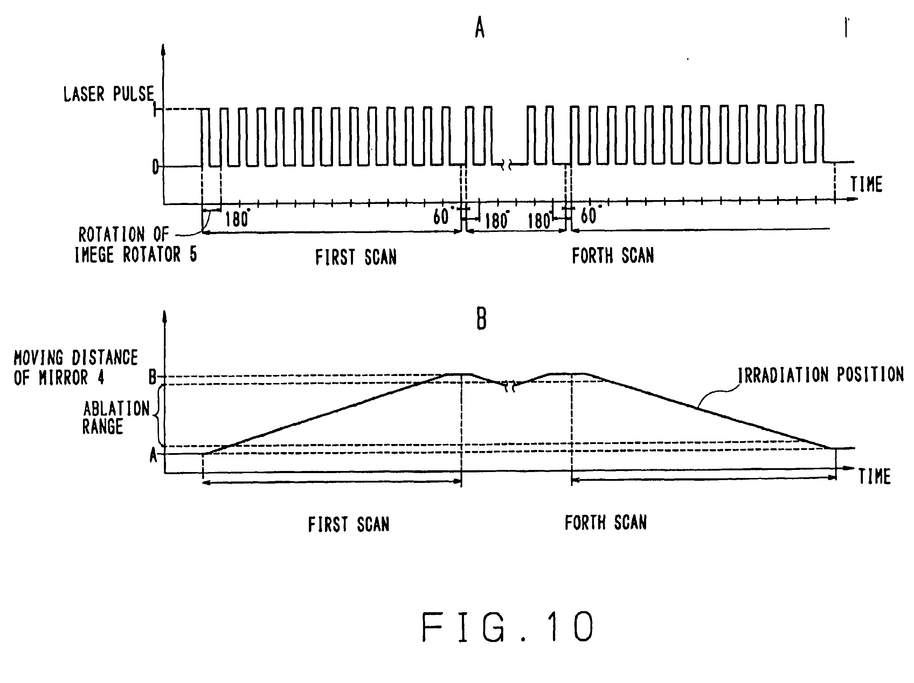

Further, as indicated in Fig. 9, in the case that the scanning direction of the laser beam is almost the opposite, shifting a position of the laser irradiation within the ablation range, more uniform ablation can be achieved (Fig. 9 shows a position of the laser irradiation 50 in the first scan in the case the scanning direction of the laser beam in Fig.9 is rotated at 120° and a position of the laser irradiation 51 in the forth scan). For example, when a position of the laser irradiation shifts as shown in Fig.9, movement of the mirror 4 in the first and forth scan can be varied as indicated in Fig. 10. In the case that the scanning direction of the laser beam is rotated at 133°, the laser irradiation allows shifting in the first scan and the forth scan. In case that scan is overlapped in the same direction, an irradiation position permits shifting.

Completing the laser irradiation for the determined number of scans, the laser irradiation is performed even if the foot switch 26 is kept pushing. After confirmation of ablation condition of the eye E, the examinee completes surgery.

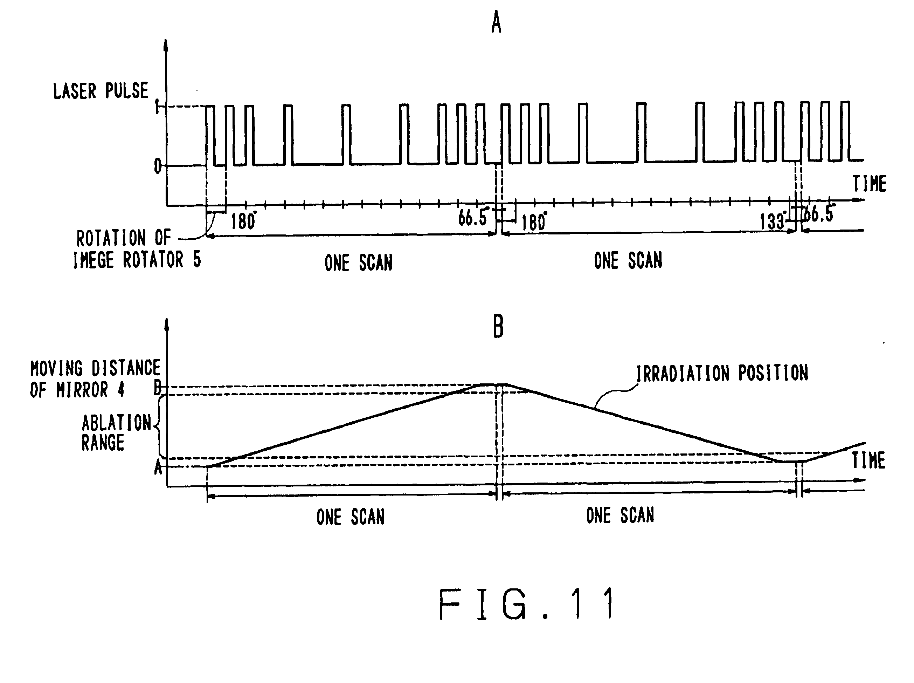

In the present embodiment, the insufficient ablation depth in the peripheral portion is prevented by varying the interval of laser pulses and rotating the scanning direction at a predetermined angle. In this case, the interval of laser pulses after a start and before completion of scanning in one scan is made rapid (short) (see Fig. 11A) relative to the moving mirror 4 (see Fig. 11B) at the fixed speed. The number of the laser pulses is increased (the irradiation interval is frequent) in the peripheral portion. As described before, rotating the scanning direction to be overlapped the second, third and fourth scan, the ablation depth can be generally uniform in the center and the peripheral portion. In the case the laser beam with successive oscillation, not pulsed oscillation, the interval of the laser emission may be changed by a shutter and the like.

Variation of the moving speed of the mirror 4 in one scan and of the interval of the laser pulses may prevent the insufficient ablation depth.

In the present embodiment, operation of the apparatus in phototherapeutic keratectomy (PTK) is described. The present invention can be applied to photorefractive keratectomy (PRK) which varies curvature of the cornea by ablating the cornea with the laser irradiation.

In the present invention, for sake of easy explanation, the laser irradiation is gradually more frequently irradiated from the center portion to the peripheral portion. The present invention is not limited to such and included similar technique. For example, according to the experiment with using removed pig's eyes by the present inventor, the laser irradiation in a peripheral portion is frequent compared to a center portion of the ablation range. The whole ablation range can be ablated uniformly by the coarse laser irradiation in outermost peripheral portion compared to near-center portion.

In the present embodiment, the laser irradiation by a scanning system is described. In the case of the flying spot system, the laser is irradiated more frequently at the peripheral portion compared to a center portion of the ablation range.

## Claims

1. A corneal surgery apparatus for ablating a cornea (Ec) of a patient's eye (E) with a laser beam, the apparatus comprising:
laser irradiation means including:
a laser light source (2); and
an irradiation optical system (3-6,8) for guiding the laser beam emitted from the laser light source (2) and irradiating the cornea with the laser beam;
input means (25) for inputting required surgery conditions which determines a required shape of the cornea (Ec) after ablation; and
control means (20) which controls the laser irradiation means (2,3-6,8) based on the inputted surgery conditions,
**characterized in that**
the control means (20) controls the laser irradiation means (2, 3-6, 8) such that the laser beam has a non-uniform irradiation frequency within an ablation area of the cornea (Ec).

2. The corneal surgery apparatus according to claim 1, wherein the control means (20) controls the laser irradiation means (2,3-6,8) such that the irradiation frequency in a peripheral portion of the ablation area is higher than the irradiation frequency in a center portion of the ablation area.

3. The corneal surgery apparatus according to claim 1 or 2, wherein the laser irradiation means (2,3-6,8) comprises scanning means (3-5,21,22) for scanning the laser beam in a predetermined direction within the ablation area.

4. The corneal surgery apparatus according to claim 3, wherein the laser light source (2) includes a laser light source emitting the laser beam whose energy intensity distribution shows non-uniform energy intensity distribution; and
the scanning means (3-5,21,22) scans the laser beam in a direction of the non-uniform intensity distribution.

5. The corneal surgery apparatus according to claim 3 or 4, wherein the control means (20) controls the scanning means (3-5,21) to vary a scanning speed of the laser beam in one scan.

6. The corneal surgery apparatus according to any of claims 3 to 5, wherein the laser light source (2) includes a laser light source which oscillates the pulsed laser beam at predetermined intervals; and
the control means (20) controls the scanning means (3-5,22) to vary a scanning speed of the laser beam relative to the pulse intervals.

7. The corneal surgery apparatus according to claim 5 or 6, wherein the control means (20)controls the scanning means (3-5,21) to slow down the scanning speed in a peripheral portion of the ablation area relative to the scanning speed in a center portion of the ablation area.

8. The corneal surgery apparatus according to any of claims 3 to 7, wherein the control means (20) controls laser emission to vary emission intervals of the laser beam in one scan.

9. The corneal surgery apparatus according to claim 8, wherein the control means (20) controls the laser emission to shorten the emission intervals in a peripheral portion of the ablation area relative to the emission intervals in a center portion of the ablation area.

10. The corneal surgery apparatus according to any of claims 3 to 9, wherein the laser irradiation means (2,3-6,8) comprises rotation means (22) which rotates a scanning direction of the laser beam with the scanning means (3-5); and
the control means (20) controls situations of laser emission and rotation by the rotation means (22) in a manner to rotate the scanning direction at a predetermined angle after every completion of one each scan.

11. The corneal surgery apparatus according to claim 10, wherein the rotation means (22) includes an image rotator (5) which rotates in a predetermined direction and at a predetermined speed;
the control means (20) shifts timing of the laser emission relative to the rotation of the image rotator (5) after completion of one each scan rotates the scanning direction at a predetermined angle.

12. The corneal surgery apparatus according to any of claims 3 to 11, wherein the control means (20) controls situations of a scan with the scanning means and a laser emission in a manner to shift an irradiation position of the laser beam on the cornea (Ec) relative to an irradiation position of a former scan in case that at least either the scanning direction of the laser beam with the scanning means (3-5,21,22) is generally the same or opposite direction relative to the former scan.

13. The corneal surgery apparatus according to claim 12, wherein the control means (20) controls the scanning means (3-5,21,22) to shift the irradiation position of the laser beam relative to the irradiation position in the former scanning.

## Patentansprüche

1. Hornhautoperationsvorrichtung zur Ablation einer Hornhaut (Ec) eines Auges (E) eines Patienten mit einem Laserstrahl, wobei die Vorrichtung umfasst:
- ein Laserbestrahlungsmittel mit:
- einer Laserlichtquelle (2); und
- einem optischen Bestrahlungssystem (3-6, 8) zum Leiten des von der Laserlichtquelle (2) ausgesendeten Laserstrahls und Bestrahlen der Hornhaut mit dem Laserstrahl;
- ein Eingabemittel (25) zur Eingabe erforderlicher Operationsbedingungen, das eine erforderliche Form der Hornhaut (Ec) nach der Ablation bestimmt; und
- ein Steuerungsmittel (20), das das Laserbestrahlungsmittel (2, 3-6, 8) auf der Grundlage der eingegebenen Operationsbedingungen steuert;
**dadurch gekennzeichnet, dass**
das Steuerungsmittel (20) das Laserbestrahlungsmittel (2, 3-6, 8) derart steuert, dass der Laserstrahl eine nicht gleichmäßige Bestrahlungsfrequenz innerhalb eines Ablationsbereichs der Hornhaut (Ec) aufweist.

2. Hornhautoperationsvorrichtung nach Anspruch 1, wobei das Steuerungsmittel (20) das Laserbestrahlungsmittel (2, 3-6, 8) derart steuert, dass die Bestrahlungsfrequenz in einem Umfangsabschnitt des Ablationsbereichs höher als die Bestrahlungsfrequenz in einem Mittenabschnitt des Ablationsbereichs ist.

3. Hornhautoperationsvorrichtung nach Anspruch 1 oder 2, wobei das Laserbestrahlungsmittel (2, 3-6, 8) ein Scanmittel (3-5, 21, 22) zum Scannen des Laserstrahls in einer vorbestimmten Richtung innerhalb des Ablationsbereichs umfasst.

4. Hornhautoperationsvorrichtung nach Anspruch 3, wobei die Laserlichtquelle (2) eine Laserlichtquelle umfasst, die den Laserstrahl aussendet, dessen Energieintensitätsverteilung eine nicht gleichmäßige Energieintensitätsverteilung zeigt, und das Scanmittel (3-5, 21, 22) den Laserstrahl in eine Richtung der nicht gleichmäßigen Intensitätsverteilung scannt.

5. Hornhautoperationsvorrichtung nach Anspruch 3 oder 4, wobei das Steuerungsmittel (20) das Scanmittel (3-5, 21) steuert, um eine Scangeschwindigkeit des Laserstrahls in einem Scan zu verändern.

6. Hornhautoperationsvorrichtung nach einem der Ansprüche 3 bis 5, wobei die Laserlichtquelle (2) eine Laserlichtquelle umfasst, die den gepulsten Laserstrahl in vorbestimmten Intervallen oszilliert, und das Steuerungsmittel (20) das Scanmittel (3-5, 22) so steuert, dass die Scangeschwindigkeit des Laserstrahls relativ zu den Impulsintervallen verändert wird.

7. Hornhautoperationsvorrichtung nach Anspruch 5 oder 6, wobei das Steuerungsmittel (20) das Scanmittel (3-5, 21) so steuert, dass die Scangeschwindigkeit in einem Umfangsabschnitt des Ablationsbereichs relativ zu der Scangeschwindigkeit in einem Mittenabschnitt des Ablationsbereichs herabgesetzt wird.

8. Hornhautoperationsvorrichtung nach einem der Ansprüche 3 bis 7, wobei das Steuerungsmittel (20) eine Laseremission so steuert, dass Aussendeintervalle des Laserstrahls in einem Scan verändert werden.

9. Hornhautoperationsvorrichtung nach Anspruch 8, wobei das Steuerungsmittel (20) die Laseremission so steuert, dass die Aussendeintervalle in einem Umfangsabschnitt des Ablationsbereichs relativ zu den Aussendeintervallen in einem Mittenabschnitt des Ablationsbereichs verkürzt werden.

10. Hornhautoperationsvorrichtung gemäß einem der Ansprüche 3 bis 9, wobei das Laserbestrahlungsmittel (2, 3-6, 8) ein Drehmittel (22) umfasst, das eine Scanrichtung des Laserstrahls mit dem Scanmittel (3-5) dreht, und das Steuerungsmittel (20) Situationen einer Laseremission und Drehung durch das Drehmittel (22) in einer Weise steuert, dass die Scanrichtung nach Beenden eines jeweiligen Scans um einen vorbestimmten Winkel gedreht wird.

11. Hornhautoperationsvorrichtung nach Anspruch 10, wobei das Drehmittel (22) einen Bilddreher (5) umfasst, der in einer vorbestimmten Richtung und in einer vorbestimmten Geschwindigkeit dreht, und das Steuerungsmittel (20) den Zeitpunkt der Laseremission relativ zur Drehung des Bilddrehers (5) verschiebt und nach Beenden eins jeweiligen Scans die Scanrichtung um einen vorbestimmten Winkel dreht.

12. Hornhautoperationsvorrichtung nach einem der Ansprüche 3 bis 11, wobei das Steuerungsmittel (20) Situationen eines Scans mit dem Scanmittel und eine Laseremission derart steuert, dass eine Bestrahlungsposition des Laserstrahls auf der Hornhaut (Ec) relativ zu einer Bestrahlungsposition eines früheren Scans verschoben wird, falls wenigstens entweder die Scanrichtung des Laserstrahls mit dem Scanmittel (3-5, 21, 22) allgemein dieselbe oder die entgegengesetzte Richtung relativ zu dem früheren Scan ist.

13. Hornhautoperationsvorrichtung nach Anspruch 12, wobei das Steuerungsmittel (20) das Scanmittel (3-5, 21, 22) so steuert, dass die Bestrahlungsposition des Laserstrahls relativ zu der Strahlungsposition in dem früheren Scan verschoben wird.

## Revendications

1. Dispositif pour la chirurgie cornéenne pour l'ablation d'une cornée (Ec) de l'oeil d'un patient (E) avec un faisceau laser, l'appareil comprenant :
un moyen d'irradiation laser incluant :
une source (2) de lumière laser ; et
un système optique (3-6, 8) d'irradiation pour guider le faisceau laser émis par la source (2) de lumière laser et irradier la cornée avec le faisceau laser ;
un moyen d'entrée (25) pour entrer les conditions chirurgicales requises qui déterminent une forme requise de la cornée (Ec) après ablation ; et
un moyen de commande (20) qui commande le moyen d'irradiation par laser (2, 3-6, 8) suivant les conditions chirurgicales entrées,
**caractérisé en ce que**
le moyen de commande (20) commande le moyen d'irradiation par laser (2, 3-6, 8) de sorte que le faisceau laser ait une fréquence d'irradiation non uniforme dans une zone d'ablation de la cornée (Ec).

2. Dispositif pour la chirurgie cornéenne selon la revendication 1, dans lequel le moyen de commande (20) commande le moyen d'irradiation par laser (2, 3-6, 8) de sorte que la fréquence d'irradiation dans une partie périphérique de la zone d'ablation soit plus élevée que la fréquence d'irradiation dans une partie centrale de la zone d'ablation.

3. Dispositif pour la chirurgie cornéenne selon la revendication 1 ou la revendication 2, dans lequel le moyen d'irradiation par laser (2, 3-6, 8) comprend un moyen de balayage (3-5, 21, 22) pour le balayage d'un faisceau laser dans une direction prédéterminée dans la zone d'ablation.

4. Dispositif pour la chirurgie cornéenne selon la revendication 3, dans lequel la source (2) de lumière laser inclut une source de lumière laser émettant le faisceau laser dont la distribution d'intensité de l'énergie présente une distribution d'intensité de l'énergie non uniforme ; et
le moyen de balayage (3-5, 21, 22) provoque le balayage du faisceau laser dans une direction de la distribution d'intensité non uniforme.

5. Dispositif pour la chirurgie cornéenne selon la revendication 3 ou la revendication 4, dans lequel le moyen de commande (20) commande le moyen de balayage (3-5, 21) pour faire varier une vitesse de balayage du faisceau laser dans un balayage.

6. Dispositif pour la chirurgie cornéenne selon l'une quelconque des revendications 3 à 5, dans lequel la source (2) de lumière laser inclut une source de lumière laser qui fait osciller le faisceau laser pulsé à des intervalles prédéterminés ; et
le moyen de commande (20) commande le moyen de balayage (3-5, 22) pour faire varier une vitesse de balayage du faisceau laser par rapport aux intervalles de pulsation.

7. Dispositif pour la chirurgie cornéenne selon la revendication 5 ou la revendication 6, dans lequel le moyen de commande (20) commande le moyen de balayage (3-5, 21) pour ralentir la vitesse de balayage dans une partie périphérique de la zone d'ablation par rapport à la vitesse de balayage dans une partie centrale de la zone d'ablation.

8. Dispositif pour la chirurgie cornéenne selon l'une quelconque des revendications 3 à 7, dans laquelle le moyen de commande (20) commande l'émission du laser pour faire varier les intervalles d'émission du faisceau laser au cours d'un balayage.

9. Dispositif pour la chirurgie cornéenne selon la revendication 8, dans lequel le moyen de commande (20) commande l'émission de laser pour raccourcir les intervalles d'émission dans une partie périphérique de la zone d'ablation par rapport aux intervalles d'émission dans une partie centrale de la zone d'ablation.

10. Dispositif pour la chirurgie cornéenne selon l'une quelconque des revendications 3 à 9, dans lequel le moyen d'irradiation par laser (2, 3-6, 8) comprend un moyen de rotation (22) qui fait tourner une direction de balayage du faisceau laser avec le moyen de balayage (3-5); et
le moyen de commande (20) commande les situations d'émission et de rotation de laser par le moyen de rotation (22) de manière à faire tourner la direction de balayage suivant un angle prédéterminer à la fin de chaque balayage.

11. Dispositif pour la chirurgie cornéenne selon la revendication 10, dans lequel le moyen de rotation (22) inclut un rotateur d'image (5) qui tourne dans une direction prédéterminée et à une vitesse prédéterminée ;
le moyen de contrôle (20) modifie le planning d'émission de laser relativement à la rotation du rotateur d'image (5) à la fin de chaque balayage et fait tourner la direction de balayage suivant un angle prédéterminé.

12. Dispositif pour la chirurgie cornéenne selon l'une quelconque des revendications 3 à 11, dans lequel le moyen de commande (20) commande les situations d'un balayage avec le moyen de balayage et une émission de laser de manière à déplacer une position d'irradiation du faisceau laser sur la cornée (Ec) par rapport à une position d'irradiation d'un balayage précédent dans le cas où au moins la direction de balayage du faisceau laser avec le moyen de balayage (3-5, 21, 22) est généralement la même ou est la direction opposée par rapport au balayage précédent.

13. Dispositif pour la chirurgie cornéenne selon la revendication 12, dans lequel le moyen de commande (20) commande le moyen de balayage (3-5, 21, 22) pour déplacer la position d'irradiation du faisceau laser par rapport à la position d'irradiation dans le balayage précédent.
